# EUROPÄISCHE PATENTANMELDUNG

(11) **EP 3 216 480 A2**
(43) Veröffentlichungstag der Anmeldung: **13.09.2017**
(21) Anmeldenummer: 17156865.2
(22) Anmeldetag: 20.02.2017
(51) Int. Cl.: A61M 21/00, A61N 5/06, G10L 15/00, G10L 21/00, G10L 25/00

(54) **EINRICHTUNG ZUR PSYCHISCHEN SELBSTSCHULUNG**

(30) Priorität: 09.03.2016 DE 102016104316
(71) Anmelder: "Wortkampfkunst" - Kommunikationsschule für Dienstleistungen, Industrie und Handwerk e.K., 06112 Halle (Saale) (DE)
(72) Erfinder: Hajek-Glöckner, Simone, 06120 Halle (Saale) (DE)
(74) Vertreter: Schaumburg und Partner Patentanwälte mbB

(57) **Zusammenfassung**

Beschrieben ist eine Einrichtung zur psychischen Selbstschulung, umfassend ein Handgerät (10) mit einem Sprachaufzeichnungsmodul (14) zum Aufzeichnen von Sprachinformation, einem Sprachverarbeitungsmodul (16) zum Generieren kognitiv umgestalteter Sprachinformation auf Grundlage der aufgezeichneten Sprachinformation, einem Sprachwiedergabemodul (18) zur Wiedergabe der kognitiv umgestalteten Sprachinformation und einem Aktivierungsmodul (20) zum Steigern der Aufmerksamkeit eines Benutzers. Das Aktivierungsmodul (20) enthält eine Beleuchturigsvorrichtung (22), die ausgebildet ist, Beleuchtungslicht in einem vorbestimmten Wellenlängenbereich auf den Benutzer auszusenden.

## Beschreibung

Die Erfindung betrifft eine Einrichtung zur psychischen Selbstschulung.

Die Anzahl an depressiven Syndromen hat in den letzten Jahren deutlich zugenommen. Dementsprechend stark ist der mit der Behandlung dieser Erkrankungen einhergehende psychotherapeutische Aufwand gestiegen, etwa in der kognitiven Verhaltenstherapie. Darin wird unter der Annahme, dass letztlich nicht die objektive Realität, sondern die subjektive Sicht des Betrachtenden über das Verhalten entscheidet, die aktive Gestaltung des Wahrnehmungsprozesses in den Vordergrund gestellt.

Der vorliegenden Erfindung liegt die Aufgabe zugrunde, technische Mittel bereitzustellen, die es einem Erkrankten ermöglicht, in Eigenbehandlung, d. h. ohne Mitwirken eines Psychotherapeuten, wirksam gegen negative Kognitionen und Aufmerksamkeitsschwierigkeiten als depressive Symptome anzugehen.

Die Erfindung löst diese Aufgabe durch die Einrichtung nach Anspruch 1. Vorteilhafte Weiterbildungen sind in den abhängigen Ansprüchen angegeben.

Die erfindungsgemäße Einrichtung zur psychischen Selbstschulung umfasst ein Handgerät mit einem Sprachaufzeichnungsmodul zum Aufzeichnen von Sprachinformation, einem Sprachbearbeitungsmodul zum Generieren kognitiv umgestalteter Sprachinformation auf Grundlage der aufgezeichneten Sprachinformation, einem Sprachwiedergabemodul zur Wiedergabe der kognitiv umgestalteten Sprachinformation und einem Aktivierungsmodul zum Steigern der Aufmerksamkeit des Benutzers, wobei das Aktivierungsmodul eine Beleuchtungsvorrichtung enthält, die ausgebildet ist, Beleuchtungslicht in einem vorbestimmten Wellenlängenbereich auf den Benutzer auszusenden.

Die erfindungsgemäße technische Lehre beruht unter anderem auf der Erkenntnis, dass der körperliche Aktivierungszustand eines Patienten während einer psychotherapeutischen Behandlung einen für den Behandlungserfolg wichtigen Parameter darstellt. Aus diesem Grund ist das erfindungsgemäße Handgerät mit einem Aktivierungsmodul ausgestattet, dessen Funktion darin besteht, die Aufmerksamkeit des Benutzers zu steigern, falls dies für die Wirksamkeit der psychotherapeutischen Behandlung erforderlich ist. Zur Steigerung der Aufmerksamkeit sieht die Erfindung eine Lichtreizung des Benutzers vor, nämlich mittels einer in dem Aktivierungsmodul enthaltenen Beleuchtungsvorrichtung, die Beleuchtungslicht in einem vorbestimmten Wellenlängenbereich auf den Benutzer aussendet. Der Wellenlängenbereich und die Abstrahlcharakteristik des Beleuchtungslichtes sind dabei so gewählt, dass die in der menschlichen Retina vorhandenen Stäbchen mit dem Beleuchtungslicht beaufschlagt werden. Dadurch wird über Photorezeptor-/Glutamat-gesteuerte Veränderungen der in der Retina vorhandenen Bipolarzellen eine natürliche Energiefreisetzung im menschlichen Auge bewirkt, die mit einer energetischen Aktivierung innerhalb des Körpers einhergeht. Infolge dieser Aktivierung wird die Aufmerksamkeit des Benutzers zeitbegrenzt gesteigert.

Vorzugsweise sollte die Lichtreizung in abgedunkelter Umgebung erfolgen, und zwar erst nach Adaption der Augen an diese Umgebung.

Das erfindungsgemäße Handgerät weist ein Sprachaufzeichnungsmodul und ein Sprachwiedergabemodul auf. Insoweit arbeitet das Handgerät wie ein Diktiergerät, mit dem Unterschied, dass das Sprachwiedergabemodul nicht die ursprünglich aufgezeichnete Sprachinformation, sondern eine neue Sprachinformation wiedergibt, die aus der ursprünglichen Information abgeleitet wird. Hierzu ist ein Sprachbearbeitungsmodul vorgesehen, das aus der von dem Benutzer in das Handgerät eingesprochenen Sprachinformation unter Berücksichtigung eines neuen psychotherapeutischen Ansatzes eine kognitiv umgestaltete Sprachinformation generiert, die über das Sprachwiedergabemodul dem Benutzer mitgeteilt wird. Das Sprachbearbeitungsmodul ist vorzugsweise als Softwaremodul realisiert und beinhaltet insbesondere ein Spracherkennungs- und Umstrukturierungsprogramm, das nach dem vorstehend genannten Therapieansatz arbeitet. Dieser Therapieansatz geht auf die Erfinderin selbst zurück und ist in "Glöckners Modell für kognitive Restrukturierung: Anleitung und Etikette für eine erfolgreiche Kommunikation", Wortkampfkunst, 1. Auflage, 2015 im Detail erläutert. Er wird im Folgenden nur kurz zusammengefasst, um den psychotherapeutischen Kontext der vorliegenden Erfindung zu umreißen.

Der neue therapeutische Ansatz basiert auf der Annahme, dass Stimmung und Verhalten eines Menschen durch eigene kognitive Strategiepläne bestimmt werden. Die im Alltag geführten Gespräche mit anderen Personen und die eigenen Gedanken und Verhaltensweisen können durch regelmäßige Analyse auf negative Kognitionen und durch kognitive Umstrukturierung sinnvoll für eine Neuordnung klarer Zielstellungen, für eine Veränderung der selektiven Aufmerksamkeit mit zielorientierter Kommunikation und für eine Fehlervermeidung in der nächsten alltagsrelevanten Situation genutzt werden.

Das Sprachverarbeitungsmodul nutzt ein orthographisches Spracherkennungsprogramm. Dieses ist für jede Sprache anwendbar. Voraussetzung ist die Verwendung des Präsens. Mit Hilfe einer vorinstallierten grammatikalischen Sprachanalysekomponente wird die von dem Sprachaufzeichnungsmodul in Form von Diktaten aufgezeichnete Sprachinformation anhand der verwendeten negativen und positiven Verben und Adjektive je Diktat hinsichtlich der resultierenden emotionalen Belastung bewertet. Dabei werden zur Beurteilung des wahren eigenen Satzinhaltes alle verneinenden Wörter wie "nein", "nicht" etc. und Vorsilben wie "a-", "un", "de-" etc. entfernt. Es gibt dann die Möglichkeit der softwaregesteuerten Umstrukturierung nach Glöckners Modell, welche anwendbar auf dem individuellen geistigen Niveau ist, um negative Kognitionen in positive umzuwandeln. Dieses Strategiegefüge wird als "Wortomics" bezeichnet. Es besteht aus einem Zwei-Satzformat mit einem standardisierten Hauptsatz und einem untergeordneten Nebensatz. Der standardisierte Hauptsatz, welcher glaubhaft die negative Kognition wiedergibt, lautet beispielsweise: "Es wird fehlschlagen, dass...". Als Basis für den Nebensatz nimmt die Software einen von dem Benutzer ausgewählten Hauptsatz und erstellt durch die Verwendung des verbalen oder adjektivalen Antonyms einen positiven Satzanteil. Die übrigen Satzbausteine werden gleich verwendet, Hilfsverben werden gelöscht.

Durch die vorstehend erläuterte Vorgehensweise adressiert der Benutzer an sein Gehirn, unter Berücksichtigung der ursprünglich negativen Kognition im ersten Satzblock, positive Ziele mit dem zweiten Satzblock. Die kognitive Umstrukturierung sollte für eine erfolgreiche Veränderung der selektiven Wahrnehmung je nach Inhalt mehrmals wiederholt werden. Bewusste Wiederholungen können von einmal pro Tag bis einmal pro Vierteljahr variieren. Je konkreter und realistischer "Wortomics" formuliert werden, umso besser werden das Leistungslevel, die Kommunikation und die zielorientierten Alltagshandlungen. Zur Überprüfung der Aufmerksamkeit wird ein spielerischer psychometrischer Aufmerksamkeitstest für jeden Tag implementiert. Die Aufmerksamkeit zum Erreichen gesetzter Ziele könnte durch Erhöhung der selektiven Wahrnehmung auch allgemein wieder gesteigert werden.

Vorzugsweise liegt der Wellenlängenbereich des Beleuchtungslichts zwischen 550 nm und 610 nm. Die Beschränkung des Wellenlängenbereichs des Beleuchtungslichts auf diesen Wellenlängenbereich hat sich als besonders günstig herausgestellt. So sind die mit dem Beleuchtungslicht beaufschlagten Stäbchen innerhalb der menschlichen Retina in diesem Bereich hinreichend sensitiv. Zum anderen enthält dieser Wellenlängenbereich kein schädliches Blaulicht. Besonders bevorzugt ist eine Wellenlänge von etwa 580 nm.

In einer vorteilhaften Ausgestaltung umfasst die Beleuchtungsvorrichtung eine Leuchtdiode und eine Beleuchtungsoptik mit zerstreuend optischer Wirkung. Eine Leuchtdiode hat eine vergleichsweise lange Lebensdauer und verursacht lediglich eine geringe Erwärmung. Außerdem lässt sie sich umweltfreundlich entsorgen. Die Verwendung einer zerstreuenden Beleuchtungsoptik hat den Vorteil, dass das von der Beleuchtungsvorrichtung ausgesendete Beleuchtungslicht zuverlässig in diejenigen Bereiche der menschlichen Retina gelangt, in denen die Stäbchen vornehmlich angeordnet sind, nämlich in den Bereichen außerhalb der Retinamitte.

Vorzugsweise ist die Beleuchtungsoptik aus einer bikonkaven Linse gebildet. Mit einer solch bikonkaven Linse lässt sich die gewünschte Beleuchtung der Stäbchen in der Retina mit geringem technischen Aufwand zuverlässig realisieren.

In einer vorteilhaften Ausführung ist die Beleuchtungsvorrichtung ausgebildet, das Beleuchtungslicht gepulst auszusenden. Es hat sich herausgestellt, dass sich die gewünschte Lichtreizung des Benutzers durch Aussenden von Lichtimpulsen der Länge beispielhaft von 0,1 s über eine Gesamtdauer von 2 min erzielen lässt.

In einer besonders bevorzugten Ausgestaltung enthält das Handgerät eine Beleuchtungssteuerung zum Variieren der Wellenlänge des Beleuchtungslichtes innerhalb des vorbestimmten Wellenlängenbereichs. Diese Beleuchtungssteuerung weist beispielsweise ein an dem Handgerät angeordnetes Bedienelement auf, das der Benutzer betätigt, um die Wellenlänge des Beleuchtungslichtes auf einen gewünschten Wert einzustellen.

Die erfindungsgemäße Einrichtung umfasst vorzugsweise eine von dem Handgerät separat vorgesehene Graukarte zum Bestimmen der individuellen Farbwahrnehmung des Benutzers in Abhängigkeit der mittels der Beleuchtungssteuerung variierbaren Wellenlänge des auf die Graukarte ausgesendeten Beleuchtungslichts. In dieser Ausführungsform blickt der Benutzer auf die Graukarte, während diese von dem Handgerät mit dem Beleuchtungslicht beleuchtet wird. Dabei befindet sich das Handgerät in einem vorbestimmten Abstand von der Graukarte. Während der Benutzer auf die Graukarte blickt, variiert er über die Beleuchtungssteuerung die Wellenlänge des Beleuchtungslichtes, bis es eine für den Benutzer angenehme Farbe wahrnimmt. Die Wellenlänge, die bei der individuellen Farbwahrnehmung eingestellt ist, wird im Weiteren bei Bedarf zur Lichtreizung genutzt, um die Aufmerksamkeit des Benutzers zu steigern.

Die Beleuchtungssteuerung umfasst beispielsweise eine Filteranordnung, mit der sich die Wellenlänge des Beleuchtungslichts nach Wunsch einstellen lässt. So ist es beispielsweise möglich, ein drehbares Filterrad mit verschiedenen Filtersegmenten vorzusehen, die sich durch Drehen des Filterrads wahlweise in den Strahlengang des Beleuchtungslichts einschwenken lassen. In einer rein bespielhaften Ausführung weist das Filterrad sieben Filtersegmente auf, deren Filtercharakteristiken so gewählt sind, dass sie nur Beleuchtungslicht der Wellenlänge 550nm, 560nm, 570nm, 580nm, 590nm, 600nm bzw. 610nm transmittieren.

Vorzugsweise ist ferner eine von dem Handgerät separat vorgesehene Farbgraukarte zum Erfassen der individuellen Aufmerksamkeit des Benutzers vorgesehen. Eine solche Farbgraukarte weist beispielsweise zwei Dichtefelder sowie sechs Farbfelder mit definierten Farbdichten auf, die jeweils 0,05D voneinander abweichen. Blickt der Benutzer auf die Farbgraukarte und stellt fest, dass alle Farbfelder gleich gut zu sehen sind, so kann er davon ausgehen, dass er momentan über eine gute Aufmerksamkeit verfügt. In diesem Fall kann das Handgerät unmittelbar zur psychischen Selbstschulung in Betrieb genommen werden. Stellt der Benutzer dagegen fest, dass nicht alle Farbfelder gleich gut zu sehen sind, so kann er von einer gewissen Übermüdung ausgehen und das erfindungsgemäße Aktivierungsmodul zur Lichtreizung in Betrieb nehmen, bevor er mit der eigentlichen psychischen Selbstschulung beginnt.

Vorzugsweise umfasst die Beleuchtungssteuerung einen Sensor zum Erfassen der Umgebungshelligkeit und stellt die Intensität des Beleuchtungslichts in Abhängigkeit der Umgebungshelligkeit ein. Wie weiter oben erwähnt, sollte die Lichtreizung des Benutzers nach Adaption seiner Augen an eine dunkle Umgebung durchgeführt werden. Der Grund hierfür ist, dass die in der Retina vorhandenen Stäbchen dem Nachtsehen oder Dämmerungssehen, d.h. dem Sehen bei geringer Helligkeit, dienen. Die Erfassung der Umgebungshelligkeit ermöglicht es, die Intensität des Beleuchtungslichtes an den Grad der Abdunkelung so anzupassen, dass die Stäbchen in der Retina auf den Lichtreiz möglichst stark reagieren, wodurch die Aufmerksamkeit des Benutzers entsprechend gesteigert wird. Die Aktivierungsfunktion der erfindungsgemäßen Einrichtung kann so zu jeder Tages- und Nachtzeit ohne Beeinträchtigung eines individuellen biologischen Tag-/Nachtrhythmus genutzt werden.

Das Handgerät weist vorzugsweise eine Anzeige zur Visualisierung von Informationen auf. Die zu visualisierenden Informationen beinhalten beispielsweise Informationen zur interaktiven Benutzerführung. So kann auf der Anzeigevorrichtung beispielsweise ein interaktives Inhaltsverzeichnis mit verschiedenen Ordnern angezeigt werden, zwischen denen der Benutzer wechseln kann. Die Ordnerstruktur kann zur visuellen Darstellung eines Themengebietes genutzt wird. Beispielsweise können die in das Handgerät eingesprochenen Diktate in verschiedenen Ordnern gespeichert werden, die mit individuellen Überschriften versehen sind. Eine solche Ordnerstruktur könnte beispielsweise verschiedene Speicherareale für "geistig", "körperlich", "kreativ", "wirtschaftlich" und "rechtlich" repräsentieren, wobei sich ein komplettes Thema aus jeweils einer dieser Komponenten ergibt.

Das Handgerät weist in einer bevorzugten Ausführung einen Energiespeicher, z.B. einen Akkumulator oder eine Batterie auf. Ohne auf ein Stromnetz angewiesen zu sein, kann das Handgerät so in entspannter Umgebung, z.B. im Freien verwendet werden.

In einer besonders vorteilhaften Ausgestaltung umfasst die erfindungsgemäße Einrichtung einen von dem Handgerät separat vorgesehenen Haltestab, an dessen einem Ende eine erste Aufnahme zur lösbaren Anbringung des Handgeräts ausgebildet ist. Vorzugsweise ist an dem anderen Ende des Haltestabs eine zweite Aufnahme zur Anlage eines Teils des Kopfes des Benutzers ausgebildet. Möchte der Benutzer seine Aufmerksamkeit mit Hilfe des Handgeräts steigern, so bringt er dieses an der ersten Aufnahme an, die sich an einem Ende des Haltestabs befindet. In der Anwendung wird das Handgelenk der ersten Aufnahme für das Handgerät gleichgesetzt. Anschließend richtet der Benutzer den Haltestab horizontal aus und bringt einen Teil seines Kopfes, z.B. sein Kinn, in Anlage an die zweite Aufnahme, die an dem anderen Ende des Haltestabs angeordnet ist. In diesem Zustand haben die Augen des Benutzers und das Handgerät einen durch die vordefinierten Abstand voneinander, der so gewählt ist, dass der von der Beleuchtungsvorrichtung des Handgeräts abgegebene Lichtreiz zuverlässig auch in der mobilen Anwendung die gewünschte Wirkung an der Retina des jeweiligen Auges des Benutzers erzielt, nämlich die dort vorhandenen Stäbchen in der gewünschten Weise beleuchtet.

In einer besonders bevorzugten Ausführung ist der Haltestab aus zwei Stabteilen gebildet, die zum Ändern der Stablänge entlang der Stabachse gegeneinander verschiebbar sind. In einer solch teleskopartigen Ausführung kann der Haltestab als Messstab für die individuelle Armlänge des Benutzers verwendet werden. Hierzu legt der Benutzer den Haltestab an seinen gestreckten Arm an und verschiebt die beiden Stabteile gegeneinander, bis die Stablänge gleich der Armlänge ist. Dann setzt er die beiden Stabteile mittels eines geeigneten Befestigungselementes gegeneinander fest. Dabei soll die Armlänge in etwa dem Abstand des Handgelenks des ausgestreckten Arms zu den Augen entsprechen.

Vorzugsweise ist die zweite Aufnahme zur lösbaren Anbringung der Graukarte und/oder der Farbgraukarte ausgebildet. Hierdurch ist es möglich, die Graukarte zur Bestimmung der individuellen Farbwahrnehmung des Benutzers bzw. die Farbgraukarte zur Bestimmung der Aufmerksamkeit des Benutzers in einem Abstand von dem Handgerät anzuordnen, der in etwa gleich dem Abstand ist, den die Augen des Benutzers von dem Handgerät haben, wenn sich der Benutzer zur Steigerung seiner Aufmerksamkeit der mit dem Handgerät durchgeführten Lichtreizung unterzieht.

Wird nun zur Bestimmung der individuellen Farbwahrnehmung des Benutzers die Graukarte bzw. zur Bestimmung der individuellen Aufmerksamkeit des Benutzers die Farbgraukarte an dem einen Ende und das Handgerät an dem anderen Ende des Haltestabs angebracht, so hat das Handgerät von der jeweiligen Karte einen Abstand, der in etwa gleich dem Abstand ist, den das Handgerät von den Augen des Benutzers hat, wenn dieser das Handgerät mit ausgestrecktem Arm vor seinen Augen hält, um die Lichtreizung durchzuführen.

Die Erfindung wird im Folgenden anhand der Figuren näher erläutert. Darin zeigen:
- Figur 1: ein Blockdiagramm eines Handgeräts, das Teil einer erfindungsgemäßen Einrichtung zur psychischen Selbstschulung ist, und
- Figur 2: einen Haltestab, an dessen einem Ende eine Graukarte bzw. Farbgraukarte und an dessen anderem Ende das Handgerät angeordnet ist.

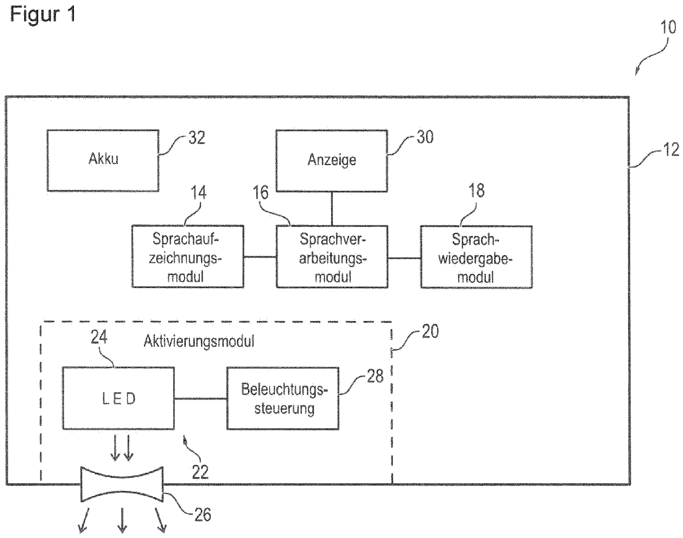

Figur 1 zeigt ein Blockdiagramm eines Handgerätes 10, das Teil einer erfindungsgemäßen Einrichtung zur psychischen Selbstschulung ist.

Das Handgerät 10 hat ein Gehäuse 12, das vorzugsweise aus einem hochwertigen Material, z.B. Titan, gefertigt ist. Das Gehäuse ist so dimensioniert, dass es ein Benutzer bequem in einer Hand halten kann.

Das Handgerät 10 enthält ein Sprachaufzeichnungsmodul 14, ein Sprachverarbeitungsmodul 16 und Sprachwiedergabemodul 18. Das Sprachaufzeichnungsmodul 16 dient in an sich bekannter Weise der Aufzeichnung der von dem Benutzer in das Handgerät 10 eingesprochenen Sprachinformation. Hierzu nutzt das Sprachaufzeichnungsmodul 14 in Figur 1 nicht explizit dargestellte Funktionskomponenten, z.B. ein Mikrophon und einen Speicher, in dem die Sprachinformation gespeichert wird.

Die von dem Benutzer in das Handgerät 10 eingesprochene und abgespeicherte Sprachinformation wird in dem Sprachverarbeitungsmodul 16 auf Grundlage des weiter oben erläuterten Umstrukturierungsmodells nach Glöckner kognitiv umgestaltet. Ziel dieser Umgestaltung ist, negative Kognitionen, die in der eingesprochenen Sprachinformation enthalten sind, in positive Kognitionen umzuwandeln. Die durch das Sprachverarbeitungsmodul 16 umgestaltete Sprachinformation wird abgespeichert. Mit Hilfe des Sprachwiedergabemoduls 18 kann die umgestaltete Sprachinformation dem Benutzer zur psychischen Selbstschulung zu Gehör gebracht werden. Hierzu verfügt das Sprachwiedergabemodul 18 über einen in Figur 1 nicht explizit gezeigten Lautsprecher.

Das Handgerät 10 enthält ferner ein Aktivierungsmodul 20 mit einer Beleuchtungsvorrichtung 22, die aus einer LED 24 und einer Beleuchtungsoptik 26 gebildet ist. Die Beleuchtungsoptik 24 ist als bikonkave Linse mit zerstreuend optischer Wirkung ausgeführt. Das Aktivierungsmodul 20 weist ferner eine Beleuchtungssteuerung 28 auf, mit der die LED 24 so angesteuert werden kann, dass die Wellenlänge und die Intensität des von der LED 24 auf die Beleuchtungsoptik 26 abgegebenen Beleuchtungslichtes variierbar sind. Der Wellenlängenbereich, innerhalb dessen sich die Wellenlänge des Beleuchtungslichtes ändern lässt, liegt beispielsweise zwischen 550 nm und 610 nm, besonders bevorzugt bei etwa 580 nm. Die Abstrahlcharakteristik der durch die LED 24 und die Beleuchtungsoptik 26 gebildeten Beleuchtungsvorrichtung 22 ist so gewählt, dass das von dem Handgerät 10 abgegebene Beleuchtungslicht innerhalb eines menschlichen Auges, das einen vorbestimmten Abstand von dem Handgerät 10 hat, vor allem die in der Retina vorhandenen Stäbchen beleuchtet.

Zur Einstellung der gewünschten Wellenlänge des Beleuchtungslichts kann die Beleuchtungssteuerung 28 beispielsweise auch eine Filteranordnung aufweisen, die sich zwischen der LED 24 und der Beleuchtungsoptik 26 befindet. Diese Filteranordnung ist z.B. als drehbares Filterrad mit verschiedenen Filtersegmenten ausgebildet, die mit Drehen des Filterrads wahlweise in den Strahlengang des Beleuchtungslichts eingebracht werden. Dabei lässt jedes der Filtersegmente nur eine vorbestimmte Wellenlänge innerhalb des auf 550nm bis 610nm beschränkten Wellenlängenbereichs durch.

Das Handgerät 10 enthält ferner eine Anzeige 30, auf der dem Benutzer verschiedene Informationen zur Darstellung gebracht werden können, beispielsweise ein Bedienmenü mit einem mehrere Ordner umfassendes Inhaltverzeichnis. Auch ist es möglich, die von dem Sprachverarbeitungsmodul 16 umgestaltete Sprachinformation dem Benutzer nicht nur über das Sprachwiedergabemodul 18 akustisch zur Kenntnis zu bringen, sondern diese Information auch auf der Anzeige 30 darzustellen.

Das Handgerät 10 enthält ferner einen Energiespeicher 32, z.B. einen Lithium-Ionen-Akkumulator, so dass es ohne externe Stromversorgung betrieben werden kann.

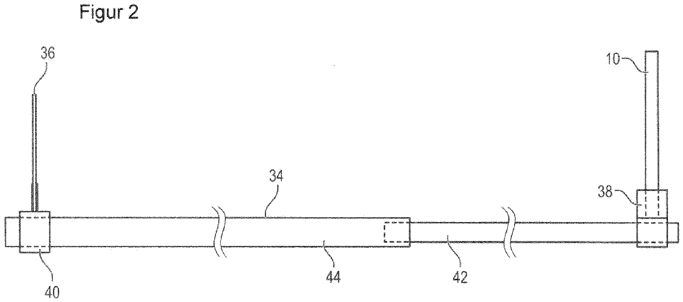

In Figur 2 ist ein Haltestab 34 gezeigt, der ebenfalls Teil der erfindungsgemäßen, zur psychischen Selbstschulung bestimmten Einrichtung ist und dazu dient, an einem Stabende das Handgerät 10 und an dem anderen Stabende eine Graukarte bzw. Farbgraukarte 36 zu halten. Zu diesem Zweck ist an dem einen Ende des Haltestabs 34 eine erste Aufnahme 38 zur lösbaren Anbringung es Handgeräts 10 und an dem anderen Ende eine zweite Aufnahme 40 zur lösbaren Anbringung der Kartte 36 vorgesehen. Die beiden Aufnahmen 38 und 40 sind beispielsweise als Steckvorrichtung ausgebildet, die in ihrer Form und Dimensionierung auf das Handgerät 10 bzw. die Karte 36 ausgelegt sind. Somit lassen sich das Handgerät 10 und die Karte 36 bei Bedarf einfach auf das jeweilige Ende des Haltestabs 34 stecken.

In dem Ausführungsbeispiel nach Figur 2 ist der Haltestab 34 teleskopartig aus zwei ineinander gesteckten und gegeneinander verschiebbaren Stabteilen 42 und 44 gebildet. Indem der Stabteil 42 in den Stabteil 34 eingeschoben oder aus diesem ausgezogen wird, lässt sich die Gesamtlänge des Haltestabs 34 nach Wunsch variieren. Insbesondere lässt sie sich der Armlänge des Benutzers anpassen, um in weiter oben beschriebener Weise bei der Bestimmung der individuellen Farbwahrnehmung oder der Erfassung der individuellen Aufmerksamkeit des Benutzers den Abstand, den die Karte 36 von dem Handgerät 10 hat, so einzustellen, dass dieser dem Abstand des Handgeräts 10 von den Augen des Benutzers entspricht, um dem Benutzer, wenn er das Handgerät 10 bei ausgestrecktem Arm in seiner Hand hält, eine individuell angepasste Lichtreizung seiner Augen zu ermöglichen.

In der vorstehend erläuterten Anwendung nutzt der Benutzer den Haltestab 34 nur zur Bestimmung seiner individuellen Farbwahrnehmung und zur Bestimmung seiner Aufmerksamkeit, nicht jedoch zur Lichtreizung. Für diese hält er das Handgerät 10 ohne Zuhilfenahme des Haltestabs 34 bei ausgestrecktem Arm in seiner Hand. Es ist jedoch ebenso möglich, den Haltestab 34 auch für die Lichtreizung einzusetzen. Insbesondere kann der Haltestab 34 so modifiziert werden, dass der Benutzer einen Teil seines Kopfes, z.B. sein Kinn, an das Ende des Haltestabs 34 anlegen kann, an dem sich die zweite Aufnahme 40 befindet. Hierzu ist die zweite Aufnahme 40 so auszubilden, dass sie nicht nur die Anbringung der Karte 36 ermöglicht, sondern auch die Anlage des Kinns des Benutzers erlaubt. Alternativ kann die Aufnahme 40 auch so ausgeführt sein, dass sie bei Bedarf von dem Haltestab 34 abgenommen und durch eine andere Aufnahme ersetzt werden kann, welche die Anlage des Kinns des Benutzers ermöglicht.

### Bezugszeichenliste

- 10: Handgerät
- 12: Gehäuse
- 14: Sprachaufzeichnungsmodul
- 16: Sprachverarbeitungsmodul
- 18: Sprachwiedergabemodul
- 20: Aktivierungsmodul
- 22: Beleuchtungsvorrichtung
- 24: LED
- 26: Beleuchtungsoptik
- 28: Beleuchtungssteuerung
- 30: Anzeige
- 32: Energiespeicher
- 34: Haltestab
- 36: Karte
- 38: Erste Aufnahme
- 40: Zweite Aufnahme
- 42,44: Stabteile

## Patentansprüche

1. Einrichtung zur psychischen Selbstschulung, umfassend ein Handgerät (10) mit:
einem Sprachaufzeichnungsmodul (14) zum Aufzeichnen von Sprachinformation,
einem Sprachverarbeitungsmodul (16) zum Generieren kognitiv umgestalteter Sprachinformation auf Grundlage der aufgezeichneten Sprachinformation,
einem Sprachwiedergabemodul (18) zur Wiedergabe der kognitiv umgestalteten Sprachinformation, und
einem Aktivierungsmodul (20) zum Steigern der Aufmerksamkeit eines Benutzers,
wobei das Aktivierungsmodul (20) eine Beleuchtungsvorrichtung (22) enthält, die ausgebildet ist, Beleuchtungslicht in einem vorbestimmten Wellenlängenbereich auf den Benutzer auszusenden.

2. Einrichtung nach Anspruch 1, **dadurch gekennzeichnet, dass** der Wellenlängenbereich des Beleuchtungslichts zwischen 550 nm und 610 nm liegt.

3. Einrichtung nach Anspruch 1 oder 2, **dadurch gekennzeichnet, dass** die Beleuchtungsvorrichtung (22) eine Leuchtdiode (24) und eine Beleuchtungsoptik (26) mit zerstreuend optischer Wirkung umfasst.

4. Einrichtung nach Anspruch 3, **dadurch gekennzeichnet, dass** die Beleuchtungsoptik (26) aus einer bikonkaven Linse gebildet ist.

5. Einrichtung nach einem der vorhergehenden Ansprüche, **dadurch gekennzeichnet, dass** die Beleuchtungsvorrichtung (22) ausgebildet ist, das Beleuchtungslicht gepulst auszusenden.

6. Einrichtung nach einem der vorhergehenden Ansprüche, **dadurch gekennzeichnet, dass** das Handgerät (10) eine Beleuchtungssteuerung (28) zum Variieren der Wellenlänge des Beleuchtungslichtes innerhalb des vorbestimmten Wellenlängenbereichs enthält.

7. Einrichtung nach einem der vorhergehenden Ansprüche, **dadurch gekennzeichnet, dass** die Beleuchtungssteuerung (28) einen Sensor zum Erfassen der Umgebungshelligkeit umfasst und die Intensität des Beleuchtungslichts in Abhängigkeit der Umgebungshelligkeit einstellt.

8. Einrichtung nach Anspruch 6 oder 7, **gekennzeichnet durch** eine von dem Handgerät (10) separat vorgesehene Graukarte (36) zum Bestimmen der individuellen Farbwahrnehmung des Benutzers in Abhängigkeit der mittels der Beleuchtungssteuerung variierbaren Wellenlänge des auf die Graukarte (36) ausgesendeten Beleuchtungslichts.

9. Einrichtung nach einem der vorhergehenden Ansprüche, **gekennzeichnet durch** eine von dem Handgerät (10) separat vorgesehene Farbgraukarte (36) zum Erfassen der individuellen Aufmerksamkeit des Benutzers.

10. Einrichtung nach einem der vorhergehenden Ansprüche, **dadurch gekennzeichnet, dass** das Handgerät (10) eine Anzeige (30) zur Visualisierung von Informationen aufweist.

11. Einrichtung nach einem der vorhergehenden Ansprüche, **dadurch gekennzeichnet, dass** das Handgerät (10) einen Energiespeicher (32) aufweist.

12. Einrichtung nach einem der vorhergehenden Ansprüche, **gekennzeichnet durch** einen von dem Handgerät (10) separat vorgesehenen Haltestab (34), an dessen einem Ende eine erste Aufnahme (38) zur lösbaren Anbringung des Handgerätes (10) ausgebildet ist.

13. Einrichtung nach Anspruch 12, **dadurch gekennzeichnet, dass** an dem anderen Ende das Haltestabs (34) eine zweite Aufnahme (40) angeordnet ist, die zur Anlage eines Teils des Kopfes des Benutzers ausgebildet ist.

14. Einrichtung nach Anspruch 13, **dadurch gekennzeichnet, dass** die zweite Aufnahme (40) zur lösbaren Anbringung der Graukarte und/oder der Farbgraukarte ausgebildet ist.

15. Einrichtung nach Anspruch 12, 13 oder 14, **dadurch gekennzeichnet, dass** der Haltestab (34) aus zwei Stabteilen (42, 44) gebildet ist, die zum Ändern der Stablänge entlang der Stabachse gegeneinander verschiebbar sind.
